# EUROPÄISCHE PATENTSCHRIFT

(11) **EP 0 552 631 B1**
(45) Veröffentlichungstag und Bekanntmachung des Hinweises auf die Patenterteilung: **24.09.1997**
(21) Anmeldenummer: 93100273.7
(22) Anmeldetag: 11.01.1993
(51) Int. Cl.: C07D 233/06, C07D 233/10, C07D 235/02, A61K 31/415

(54) **Alpha-Aryl-alpha-hydroxy-beta-imidazolinylpropionsäureamide**
Alpha-aryl-alpha-hydroxy-beta-imidazolinylpropionamides
Amides de l'acide alpha-aryl-alpha-hydroxy-bêta-imidazolinyl propionique

(30) Priorität: 23.01.1992 DE 4201709
(43) Veröffentlichungstag der Anmeldung: 28.07.1993
(73) Patentinhaber: BAYER AG, 51368 Leverkusen (DE)
(72) Erfinder: Draber, Wilfried, Dr., W-5600 Wuppertal (DE); Bischoff, Hilmar, Dr., W-5600 Wuppertal (DE)

(56) Entgegenhaltungen:
- WO-A-91/00862
- DE-A- 2 623 377
- FR-A- 2 355 501
- US-A- 4 136 188

## Beschreibung

Die vorliegende Erfindung betrifft neue α-Aryl-α-hydroxy-β-imidazolinyl-propionsäureamide, ein Verfahren zu ihrer Herstellung und ihre Verwendung in Arzneimitteln.

Die erfindungsgemäßen neuen Verbindungen sind durch die folgende allgemeine Formel (I) gekennzeichnet 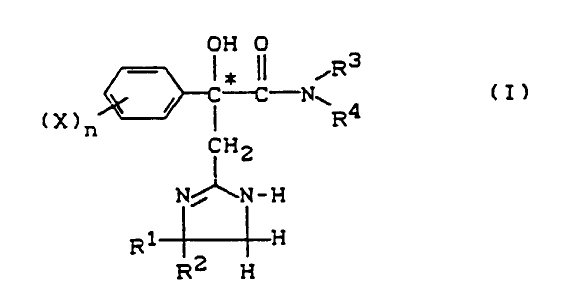 in welcher
- n: für die Zahlen 0, 1, 2 oder 3 steht,
- R¹ und R²: gleich oder verschieden sind und einzeln für Wasserstoff oder C₁-C₆-Alkyl oder zusammen für C₄-C₆-Alkandiyl stehen,
- R³: für Wasserstoff oder C₁-C₆-Alkyl steht,
- R⁴: für Wasserstoff, für gegebenenfalls durch Fluor, Chlor oder C₁-C₄-Alkoxy substituiertes C₁-C₆-Alkyl, für jeweils gegebenenfalls durch Fluor, Chlor, Brom oder C₁-C₄-Alkyl substituiertes C₃-C₇-Cycloalkyl oder C₃-C₇-Cycloalkyl-C₁-C₄-alkyl, für jeweils gegebenenfalls substituiertes Phenyl, Naphthyl, Phenyl-C₁-C₄-alkyl oder Naphthyl-C₁-C₄-alkyl steht (wobei die möglichen Substituenten vorzugsweise ausgewählt sind aus der Reihe Fluor, Chlor, Brom, Cyano, Nitro, jeweils gegebenenfalls durch Fluor und/oder Chlor substituiertes C₁-C₄-Alkyl, C₁-C₄-Alkoxy, C₁-C₄-Alkylthio, C₁-C₄-Alkylsulfinyl, C₁-C₄-Alkylsulfonyl), oder
- R³ und R⁴: zusammen mit dem Stickstoffatom, an das sie gebunden sind, einen gegebenenfalls einfach bis dreifach durch C₁-C₄-Alkyl substituierten, gesättigten oder ungesättigten, fünf- bis siebengliedrigen Stickstoffheterocyclus bilden, der gegebenenfalls ein weiteres Heteroatom, wie Sauerstoff oder Stickstoff enthält, und
- X: für Fluor, Chlor, Brom oder für jeweils gegebenenfalls durch Fluor und/oder Chlor substituiertes C₁-C₄-Alkyl oder C₁-C₄-Alkoxy steht.

Insbesondere betrifft die vorliegende Erfindung Verbindungen der Formel (I), in welcher
- n: für die Zahlen 0, 1 oder 2 steht,
- R¹ und R²: gleich oder verschieden sind und einzeln für Wasserstoff, Methyl, Ethyl, Propyl, Isopropyl, Butyl, Isobutyl oder sec-Butyl stehen oder zusammen für Butan-1,4-diyl (Tetramethylen) oder Pentan-1,5-diyl (Pentamethylen) stehen,
- R³: für Wasserstoff, Methyl, Ethyl, Propyl, Isopropyl, Butyl, Isobutyl oder sec-Butyl steht,
- R⁴: für Wasserstoff, Methyl, Ethyl, Propyl, Isopropyl, Butyl, Isobutyl, sec-Butyl, tert-Butyl, Pentyl, Isopentyl, sec-Pentyl, tert-Pentyl, Hexyl, Cyclopentyl, Cyclohexyl, Cyclopentylmethyl, Cyclohexylmethyl, für jeweils gegebenenfalls substituiertes Phenyl, Benzyl oder Phenylethyl steht (wobei die möglichen Substituenten insbesondere ausgewählt sind aus der Reihe Fluor, Chlor, Methyl, Ethyl, Trifluormethyl, Methoxy, Ethoxy, Difluormethoxy, Trifluormethoxy), oder
- R³ und R⁴: zusammen mit dem Stickstoffatom, an das sie gebunden sind, für jeweils gegebenenfalls einfach bis dreifach durch Methyl und/oder Ethyl substituiertes Pyrrolidinyl, Piperidinyl, Morpholinyl oder Piperazinyl stehen, und
- X: für Fluor, Chlor, Methyl, Ethyl, Trifluormethyl, Methoxy, Ethoxy, Difluormethoxy oder Trifluormethoxy steht.

Alkyl, auch in Verbindungen mit Heteroatomen, wie z. B. in Alkoxy, ist jeweils geradkettig oder verzweigt.

Die Erfindung betrifft auch physiologisch verträgliche Säureaddukte der Verbindungen der Formel (I).

Die Verbindungen der Formel (I) besitzen mindestens ein asymmetrisch substituiertes (mit ∗ markiertes) Kohlenstoffatom und können deshalb in verschiedenen stereoisomeren Formen anfallen. Die Erfindung betrifft sowohl die einzelnen möglichen Stereoisomeren als auch die verschiedenen möglichen Gemische dieser Stereoisomeren.

Bevorzugte erfindungsgemäße Verbindungen sind auch Additionsprodukte aus Säuren und denjenigen Verbindungen der Formel (I), in denen n, R¹, R², R³, R⁴ und X diejenigen Bedeutungen haben, die im Zusammenhang mit der Beschreibung der erfindungsgemäßen Verbindungen bereits vorzugsweise für den Index und die Substituenten genannt wurden.

Zu den Säuren, die addiert werden können, gehören vorzugsweise Halogenwasserstoffsäuren, wie z.B. Chlorwasserstoffsäure und Bromwasserstoffsäure, ferner Phosphorsäure, Essigsäure, Ameisensäure, Maleinsäure, Weinsäure, Citronensäure, Oxalsäure, Salicylsäure, Sorbinsäure, Milchsäure, Schwefelsäure, Methansulfonsäure und p-Toluolsulfonsäure.

Man erhält die erfindungsgemäßen Verbindungen der Formel (I), wenn man
Methylimidazoline der allgemeinen Formel (II) 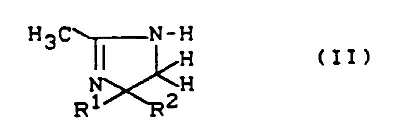 in welcher
- R¹ und R²: die oben angegebene Bedeutung haben,
mit Phenylglyoxylsäureamiden der allgemeinen Formel (III) 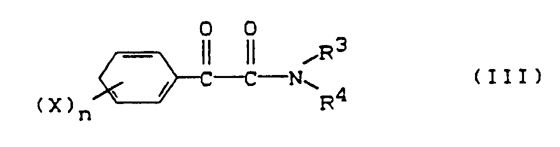 in welcher
- n, R³, R⁴ und X: die oben angegebene Bedeutung haben,
gegebenenfalls in Gegenwart eines Verdünnungsmittels und gegebenenfalls in Gegenwart eines basischen Katalysators umsetzt.

Verwendet man beim erfindungsgemäßen Verfahren zur Herstellung der Verbindungen der Formel (I) beispielsweise 2-Methyl-4-propyl-2-imidazolin und Phenylglyoxylsäuredimethylamid als Ausgangsstoffe, so kann der Reaktionsablauf durch das folgende Formelschema skizziert werden: 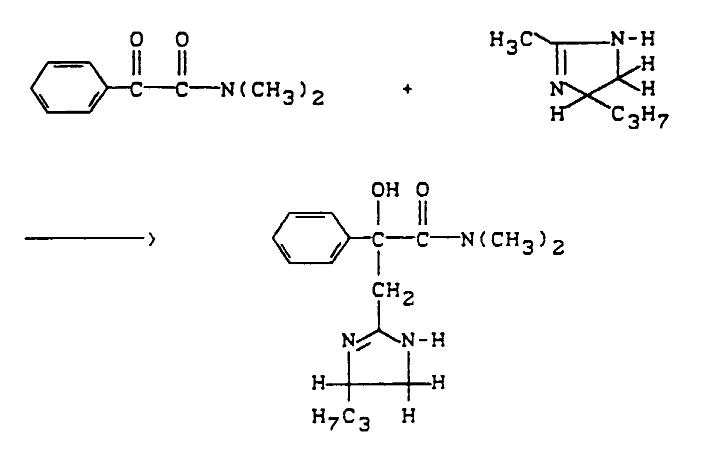 Die beim erfindungsgemäßen Verfahren zur Herstellung von Verbindungen der Formel (I) als Ausgangsstoffe zu verwendenden Methylimidazoline sind durch die Formel (II) allgemein definiert.

In Formel (II) haben R¹ und R² vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für R¹ und R² angegeben wurden.

Die Ausgangsstoffe der Formel (II) sind bekannt und/oder können nach an sich bekannten Verfahren hergestellt werden (vgl. J. Am. Chem. Soc. 71 (1949), 2530-2531).

Die beim erfindungsgemäßen Verfahren weiter als Ausgangsstoffe zu verwendenden Phenylglyoxylsäureamide sind durch die Formel (III) allgemein definiert.

In Formel (III) haben n, R³, R⁴ und X vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für n, R³, R⁴ und X angegeben wurden.

Die Ausgangsstoffe der Formel (III) sind bekannt und/oder können nach an sich bekannten Verfahren hergestellt werden (vgl. DE-OS 2614240; EP-A 53408; J. Am. Chem. Soc. 107 (1985), 3235-3245; Tetrahedron 33 (1977), 2437-2440; J. Org. Chem. 52 (1987), 4978-4984; Herstellungsbeispiele).

Das erfindungsgemäße Verfahren zur Herstellung der neuen Verbindungen der Formel (I) wird vorzugsweise unter Verwendung von Verdünnungsmitteln durchgeführt. Als Verdünnungsmittel kommen dabei praktisch alle inerten organischen Lösungsmittel infrage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl-, Methyl-tert-butyl-, Dipropyl- und Dibutylether, Glykoldimethylether und Diglykoldimethylether, Tetrahydrofuran und Dioxan, Ketone wie Aceton, Methyl-ethyl-, Methyl-isopropyl- und Methyl-isobutylketon, Ester wie Essigsäuremethylester und -ethylester, Nitrile wie z. B. Acetonitril und Propionitril, Amide wie z. B. Dimethylformamid, Dimethylacetamid und N-Methyl-pyrrolidon sowie Dimethylsulfoxid, Tetramethylensulfon und Hexamethylphosphorsäuretriamid.

Das erfindungsgemäße Herstellungsverfahren wird vorzugsweise unter Verwendung eines basischen Katalysators durchgeführt. Als Katalysatoren kommen vorzugsweise organische, basische Stickstoffverbindungen in Betracht. Als Beispiele hierfür seien genannt: Trimethylamin, Triethylamin, Diisopropylamin, Diisobutylamin, Dicyclohexylamin, Ethyldiisopropylamin, N,N-Dimethyl-benzylamin, Pyridin, 4-Dimethylaminopyridin, Pyrrolidin, Piperidin und 4-Methyl-piperidin.

Die Reaktionstemperaturen können bei dem erfindungsgemäßen Verfahren in einem größeren Bereich variiert werden. Im allgemeinen arbeitet man bei Temperaturen zwischen 0 °C und 150 °C, vorzugsweise bei Temperaturen zwischen 10 °C und 100 °C.

Das erfindungsgemäße Verfahren wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel in Gegenwart eines basischen Katalysators durchgeführt, und das Reaktionsgemisch wird mehrere Stunden bei der jeweils erforderlichen Temperatur gerührt. Die Aufarbeitung erfolgt bei dem erfindungsgemäßen Verfahren jeweils nach üblichen Methoden (vgl. die Herstellungsbeispiele).

Die erfindungsgemäßen Verbindungen der allgemeinen Formel (I) zeigen ein wertvolles pharmakologisches Wirkspektrum.

Bei nur geringer Kreislaufwirkung senken sie den Blutzucker und können so zur Behandlung des Diabetes eingesetzt werden.

Die erfindungsgemäßen Verbindungen können in bekannter Weise in die üblichen Formulierungen überführt werden, wie Tabletten, Kapseln, Dragees, Pillen, Granulate, Aerosole, Sirupe, Emulsionen, Suspensionen und Lösungen, unter Verwendung inerter, nichttoxischer, pharmazeutisch geeigneter Trägerstoffe oder Lösungsmittel. Hierbei soll die therapeutisch wirksame Verbindung jeweils in einer Konzentration von etwa 0,5 bis 90 Gew.-% der Gesamtmischung vorhanden sein, d.h. in Mengen, die ausreichend sind, um den angegebenen Dosierungsspielraum zu erreichen.

Die Applikation erfolgt in üblicher Weise, vorzugsweise oral oder parenteral, insbesondere perlingual oder intravenös.

Im allgemeinen hat es sich als vorteilhaft erwiesen, bei oraler Applikation Mengen von etwa 0,01 bis 200 mg/kg, vorzugsweise 0,1 bis 50 mg/kg Körpergewicht zur Erzielung wirksamer Ergebnisse zu verabreichen.

Trotzdem kann es gegebenenfalls erforderlich sein, von den genannten Mengen abzuweichen, und zwar in Abhängigkeit vom Körpergewicht des Versuchstieres (bei der Austestung am Tiermodell) bzw. der Art des Applikationsweges, aber auch aufgrund der Tierart und deren individuellem Verhalten gegenüber dem Medikament bzw. der Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehrere Einzelgaben über den Tag zu verteilen. Für die Applikation in der Humanmedizin ist der gleiche Dosierungsspielraum vorgesehen. Sinngemäß gelten hierbei auch die obigen Ausführungen.

### Herstellungsbeispiele:

### Beispiel 1

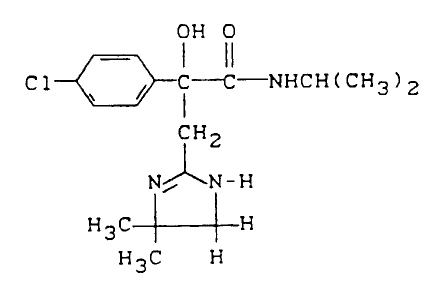

Eine Lösung von 11,2 g (0,10 Mol) 2,4,4-Trimethyl-2-imidazolin in 60 ml Diethylether/Methylenchlorid (Vol. 5:1) wird bei 20°C tropfenweise unter Rühren zu einer Mischung aus 22,55 g (0,10 Mol) (4-Chlor-phenyl)-glyoxylsäure-isopropylamid, 200 ml Diethylether und 0,2 g Piperidin gegeben. Das Reaktionsgemisch wird 2 Stunden unter Rühren zum Rückfluß erhitzt und anschließend eingeengt. Der Rückstand wird aus Methyl-tert-butylether kristallin erhalten und durch Absaugen isoliert.

Man erhält 26,9 g (80% der Theorie) 2-(4-Chlor-phenyl)-2-hydroxy-3-(4,4-dimethyl-2-imidazolinyl)-propansäureisopropylamid vom Schmelzpunkt 118°C.

Analog Beispiel 1 und entsprechend der allgemeinen Beschreibung des erfindungsgemäßen Herstellungsverfahrens können beispielsweise auch die in der nachstehenden Tabelle 1 aufgeführten Verbindungen der Formel (I) hergestellt werden.

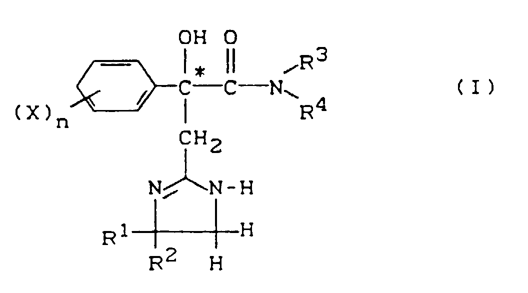

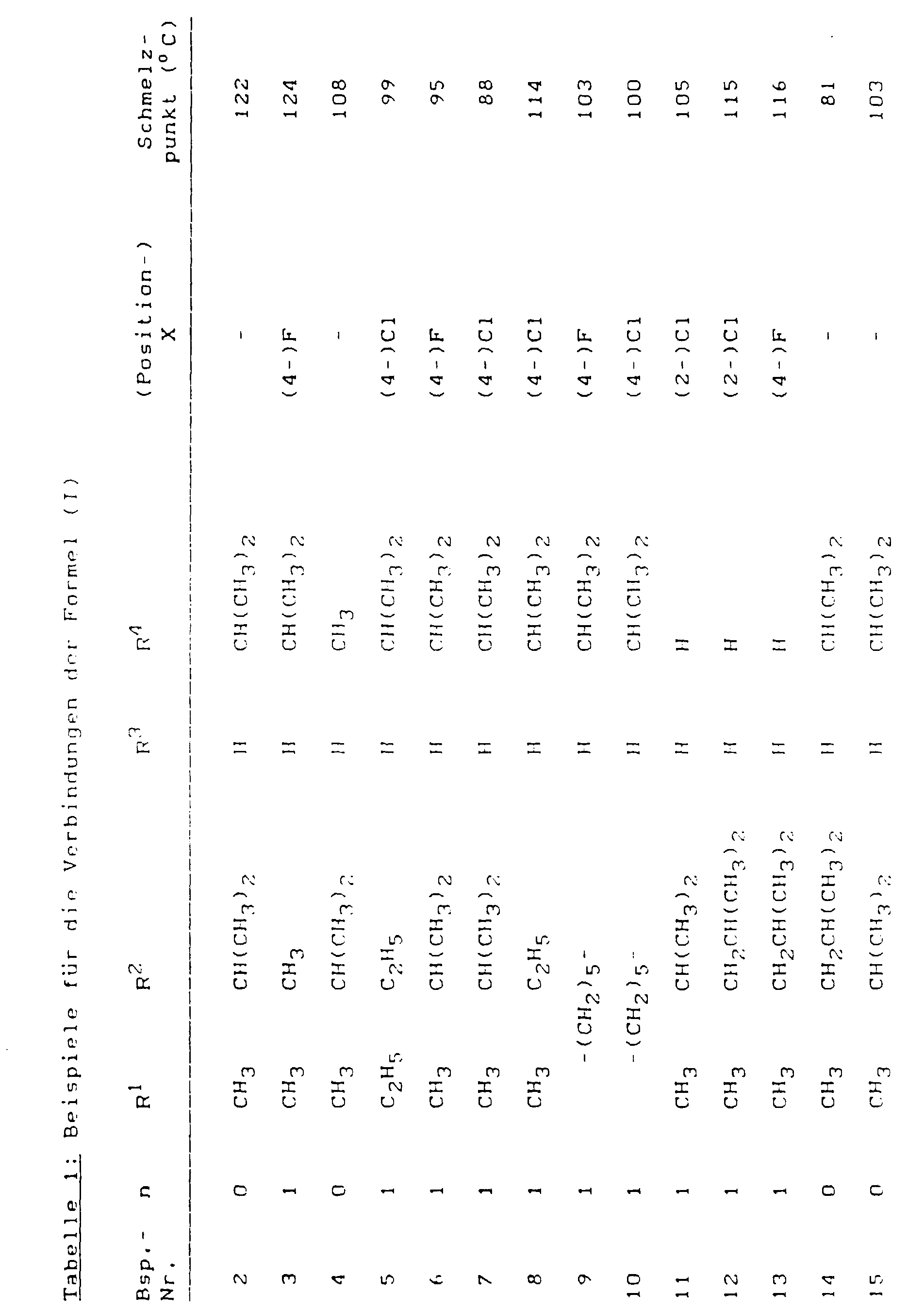

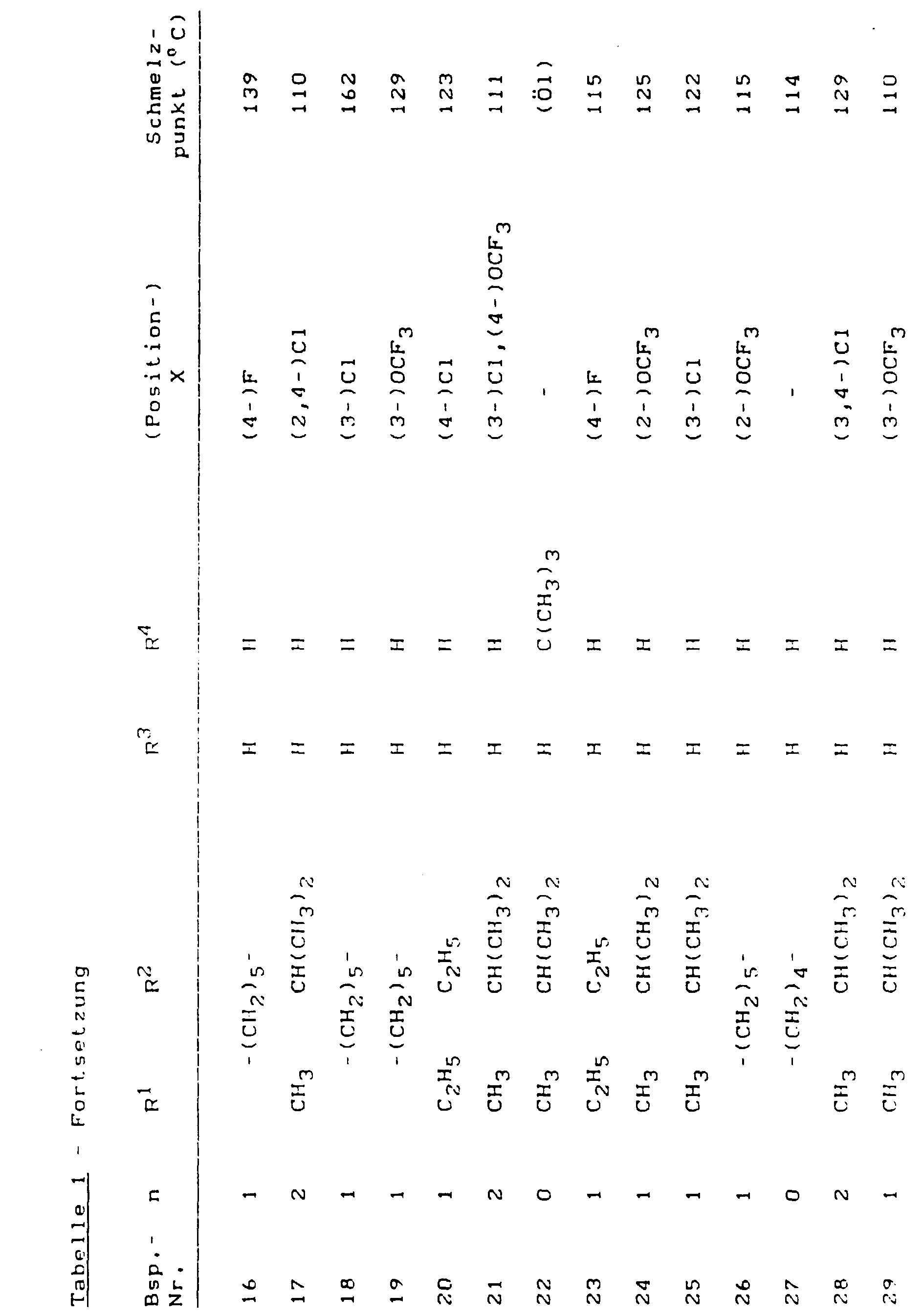

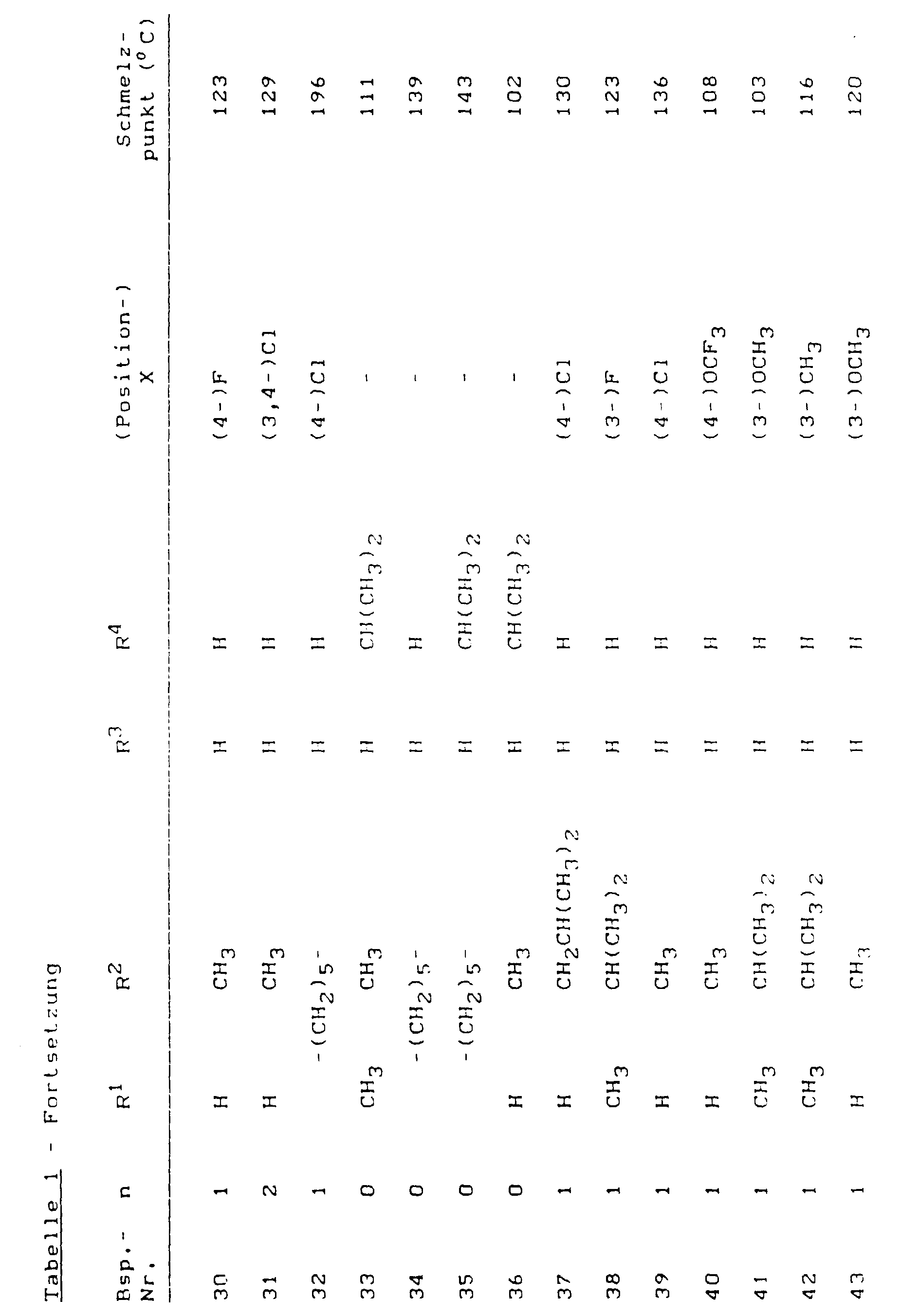

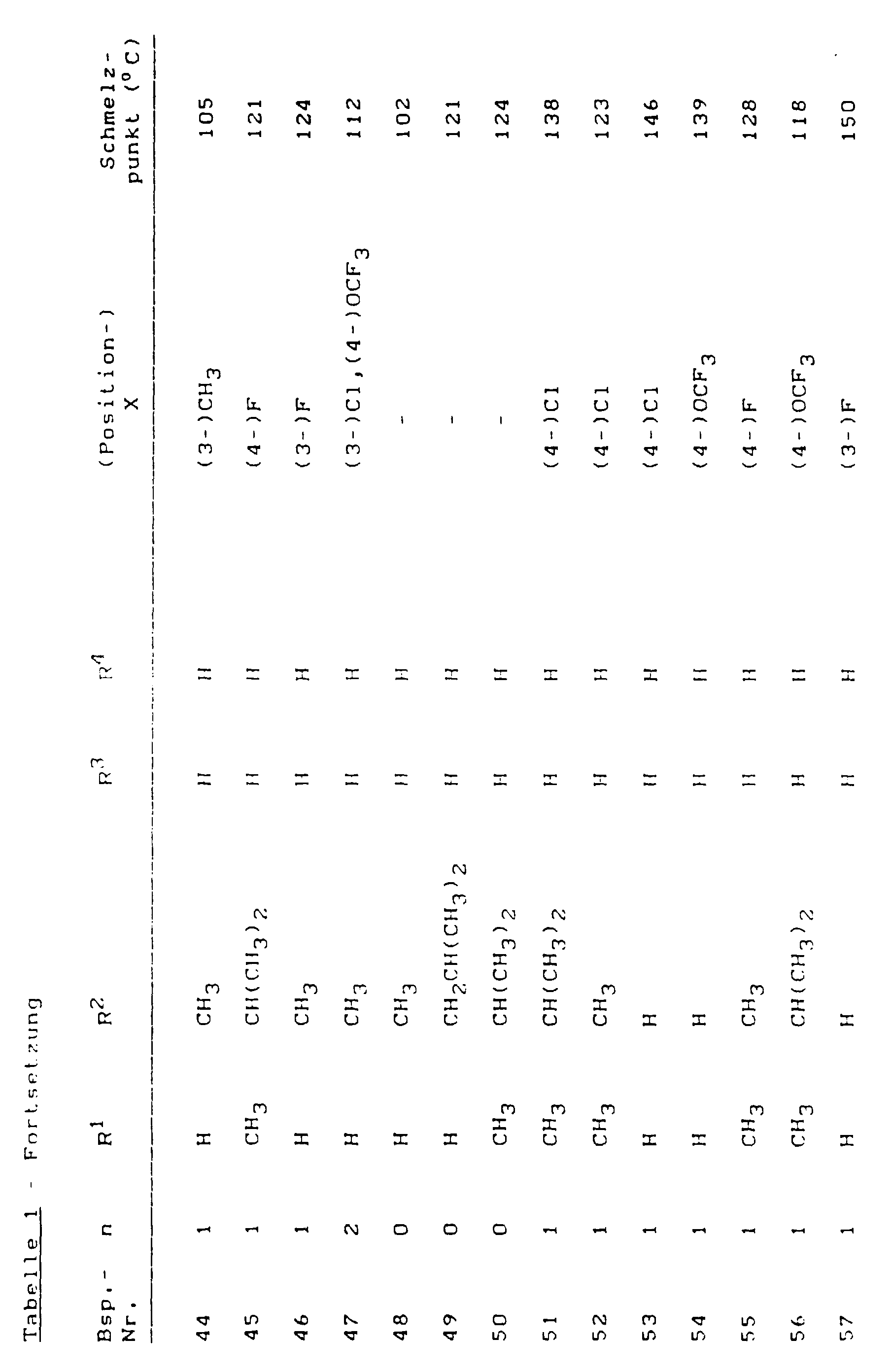

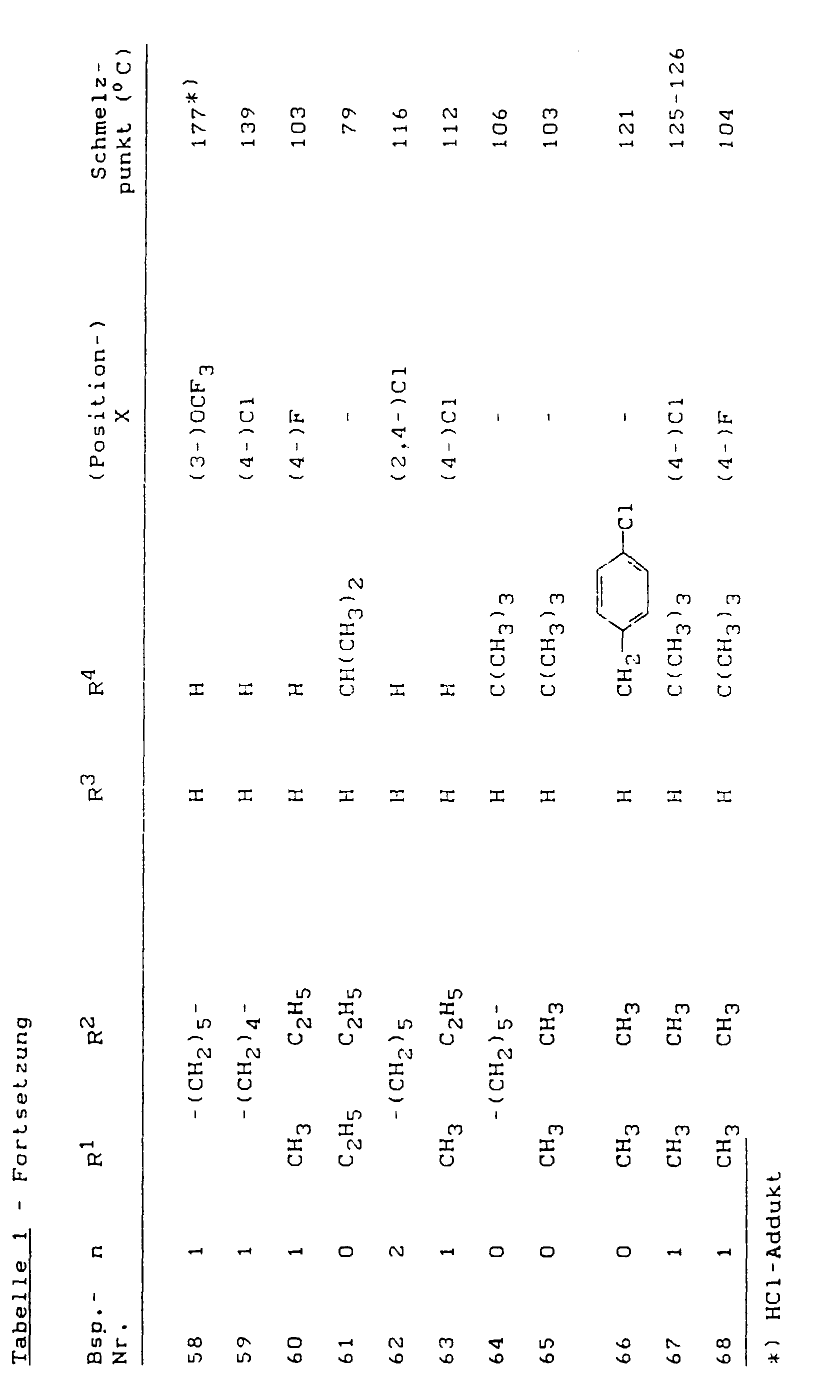

### Ausgangsstoffe der Formel (III)

### Beispiel (III-1)

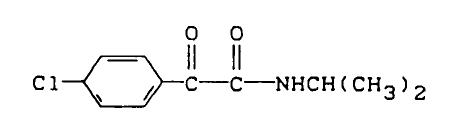

Eine Lösung von 6,9 g (117 mMol) Isopropylamin in 50 ml Methyl-tert-butylether wird bei 20°C unter Rühren zu einer Mischung aus 23,3 g (117 mMol) (4-Chlor-phenyl)-glyoxylsäure-methylester und 200 ml Methyl-tert-butylether tropfenweise gegeben. Das Reaktionsgemisch wird 20 Stunden bei 20°C gerührt, dann im Wasserstrahlvakuum eingeengt und anschließend im Ölpumpenvakuum entgast.

Man erhält 21,3 g (81% der Theorie) (4-Chlor-phenyl)-glyoxylsäure-isopropylamid vom Schmelzpunkt 79°C.

### Anwendungsbeispiele:

Die blutglucosesenkende Wirkung der zu untersuchenden Substanzen wurde an männlichen Wistar-Ratten mit einem Gewicht zwischen 140 und 190 g geprüft. Die Ratten wurden zu diesem Zweck 18h vor der Applikation der Substanzen gewogen, in Gruppen zu 6 Tieren eingeteilt und nüchtern gesetzt. Die zu untersuchenden Substanzen wurden direkt vor der Applikation in wäßriger 0,75%iger Traganthsuspension mit einem Ultra-Turrax suspendiert. Die Applikation der Traganthsuspension (Kontrolltiere) bzw. der in Traganth suspendierten Substanzen erfolgte mittels einer Schlundsonde.

Die Blutentnahme erfolgte bei jeder Ratte 30, 60, 120 und 240 Min. nach der Applikation aus dem retroorbitalen Venenplexus. Jeweils 30 µl Blut wurden mit einem automatischen Dilutor entnommen und mit 0,3 ml Uranylacetat (0,16%) enteiweißt. Nach der Zentrifugation wurde die Glucose im Überstand nach der Glucoseoxidase-Methode mit 4-Amino-phenazon als Farbreagenz an einem EPOS Analyzer 5060 photometrisch bestimmt. Die Auswertung der Ergebnisse erfolgte mit dem t-Test nach Student nach vorheriger Prüfung auf Homogenität der Varianzen, als Signifikanzgrenze wurde p < 0,05 gewählt.

Substanzen, die bei Ratten zu einem Zeitpunkt eine signifikante Reduktion der Blutglucosekonzentration um mindestens 10% bewirkten, verglichen mit der Kontrollgruppe, die nur Traganthsuspension erhielt, wurden als wirksam angegeben.

Die nachfolgende Tabelle A enthält die gefundenen Veränderungen der Blutglucosekonzentration in Prozent der Kontrolle.

**Tabelle A**

| Verbindung aus Herstellungsbeispiel Nr. | Abnahme der Blutglucosekonzentrationen in % der Kontrolle 30 mg/kg p.o. |
|---|---|
| 1 | 38 |
| 10 | 17 |
| 3 | 39 |
| 20 | 26 |
| 22 | 26 |
| 23 | 24 |
| 24 | 13 |
| 25 | 19 |
| 33 | 23 |
| 34 | 17 |
| 35 | 28 |
| 36 | 18 |
| 45 | 21 |
| 51 | 21 |
| 52 | 22 |
| 65 | 27 |
| 67 | 42 |
| 68 | 43 |

## Patentansprüche

1. Verbindungen der allgemeinen Formel (I) 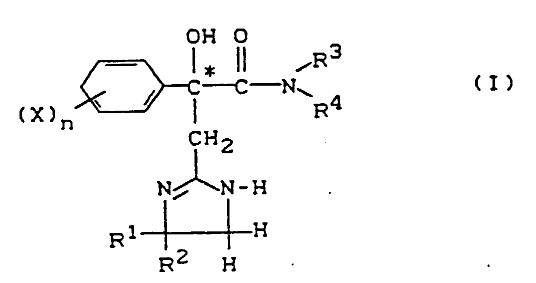 in welcher
n für die Zahlen 0, 1, 2 oder 3 steht,
R¹ und R² gleich oder verschieden sind und einzeln für Wasserstoff oder C₁-C₆-Alkyl oder zusammen für C₄-C₆-Alkandiyl stehen,
R³ für Wasserstoff oder C₁-C₆-Alkyl steht,
R⁴ für Wasserstoff, für gegebenenfalls durch Fluor, Chlor oder C₁-C₄-Alkoxy substituiertes C₁-C₆-Alkyl, für jeweils gegebenenfalls durch Fluor, Chlor, Brom oder C₁-C₄-Alkyl substituiertes C₃-C₇-Cycloalkyl oder C₃-C₇-Cycloalkyl-C₁-C₄-alkyl, für jeweils gegebenenfalls substituiertes Phenyl, Naphthyl, Phenyl-C₁-C₄-alkyl oder Naphthyl-C₁-C₄-alkyl steht (wobei die möglichen Substituenten vorzugsweise ausgewählt sind aus der Reihe Fluor, Chlor, Brom, Cyano, Nitro, jeweils gegebenenfalls durch Fluor und/oder Chlor substituiertes C₁-C₄-Alkyl, C₁-C₄-Alkoxy, C₁-C₄-Alkylthio, C₁-C₄-Alkylsulfinyl, C₁-C₄-Alkylsulfonyl), oder
R³ und R⁴ zusammen mit dem Stickstoffatom, an das sie gebunden sind, einen gegebenenfalls einfach bis dreifach durch C₁-C₄-Alkyl substituierten, gesättigten oder ungesättigten, fünf- bis siebengliedrigen Stickstoffheterocyclus bilden, der gegebenenfalls ein weiteres Heteroatom, wie Sauerstoff oder Stickstoff enthält, und
X für Fluor, Chlor, Brom oder für jeweils gegebenenfalls durch Fluor und/oder Chlor substituiertes C₁-C₄-Alkyl oder C₁-C₄-Alkoxy steht.

2. Verbindungen der Formel (I) gemäß Anspruch 1, in welcher
n für die Zahlen 0, 1 oder 2 steht,
R¹ und R² gleich oder verschieden sind und einzeln für Wasserstoff, Methyl, Ethyl, Propyl, Isopropyl, Butyl, Isobutyl oder sec-Butyl stehen oder zusammen für Butan-1,4-diyl (Tetramethylen) oder Pentan-1,5-diyl (Pentamethylen) stehen,
R³ für Wasserstoff, Methyl, Ethyl, Propyl, Isopropyl, Butyl, Isobutyl oder sec-Butyl steht,
R⁴ für Wasserstoff, Methyl, Ethyl, Propyl, Isopropyl, Butyl, Isobutyl, sec-Butyl, tert-Butyl, Pentyl, Isopentyl, sec-Pentyl, tert-Pentyl, Hexyl, Cyclopentyl, Cyclohexyl, Cyclopentylmethyl, Cyclohexylmethyl, für jeweils gegebenenfalls substituiertes Phenyl, Benzyl oder Phenylethyl steht (wobei die möglichen Substituenten insbesondere ausgewahlt sind aus der Reihe Fluor, Chlor, Methyl, Ethyl, Trifluormethyl, Methoxy, Ethoxy, Difluormethoxy, Trifluormethoxy), oder
R³ und R⁴ zusammen mit dem Stickstoffatom, an das sie gebunden sind, für jeweils gegebenenfalls ein fach bis dreifach durch Methyl und/oder Ethyl substituiertes Pyrrolidinyl, Piperidinyl, Morpholinyl oder Piperazinyl stehen, und
X für Fluor, Chlor, Methyl, Ethyl, Trifluormethyl, Methoxy, Ethoxy, Difluormethoxy oder Trifluormethoxy steht.

3. Verfahren zur Herstellung von Verbindungen der Formel (I) gemäß Anspruch 1, dadurch gekennzeichnet, daß man
Methylimidazoline der allgemeinen Formel (II) 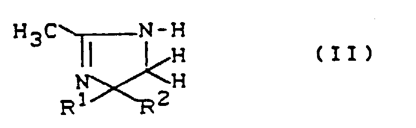 in welcher
R¹ und R² die oben angegebene Bedeutung haben, mit Phenylglyoxylsäureamiden der allgemeinen Formel (III) 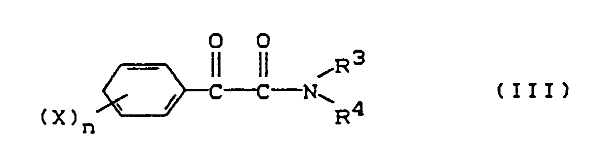 in welcher
n, R³, R⁴ und Xdie oben angegebene Bedeutung haben,
gegebenenfalls in Gegenwart eines Verdünnungsmittels und gegebenenfalls in Gegenwart eines basischen Katalysators umsetzt.

4. Arzneimittel enthaltend eine oder mehrere Verbindungen der Ansprüche 1 und 2.

5. Antidiabetika enthaltend eine oder mehrere Verbindungen der Ansprüche 1 und 2.

6. Verwendung der Verbindungen gemäß der Ansprüche 1 und 2 zur Herstellung von Arzneimitteln.

## Claims

1. Compounds of the general formula (I) 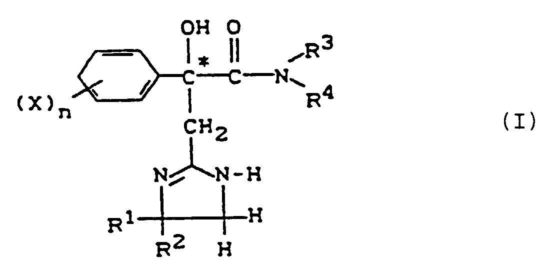 in which
n represents the numbers 0, 1, 2 or 3,
R¹ and R² are the same or different and represent individually hydrogen or C₁-C₆-alkyl or represent together C₄-C₆-alkanediyl,
R³ represents hydrogen or C₁-C₆-alkyl,
R⁴ represents hydrogen, C₁-C₆-alkyl, which is optionally substituted with fluorine, chlorine or C₁-C₄-alkoxy, C₃-C₇-cycloalkyl or C₃-C₇-cycloalkyl-C₁-C₄-alkyl each of which is optionally substituted with fluorine, chlorine, bromine or C₁-C₄-alkyl, in each case optionally substituted phenyl, naphthyl, phenyl-C₁-C₄-alkyl or naphthyl-C₁-C₄-alkyl (the possible substituents preferably being chosen from one of the following: fluorine, chlorine, bromine, cyano, nitro, or C₁-C₄-alkyl, C₁-C₄-alkoxy, C₁-C₄-alkylthio, C₁-C₄-alkylsulphinyl or C₁-C₄-alkylsulphonyl each of which is optionally substituted with fluorine and/or chlorine), or
R³ and R⁴ form, together with the nitrogen atom to which they are bound, a saturated or unsaturated, five- to seven-membered nitrogen heterocycle, which is optionally substituted once to three times with C₁-C₄-alkyl and which optionally contains a further hetero atom such as oxygen or nitrogen, and
X represents fluorine, chlorine, bromine, or C₁-C₄-alkyl or C₁-C₄-alkoxy each of which is optionally substituted with fluorine and/or chlorine.

2. Compounds of the formula (I) according to Claim 1, in which
n represents the numbers 0, 1 or 2,
R¹ and R² are the same or different and represent individually hydrogen, methyl, ethyl, propyl, isopropyl, butyl, isobutyl or sec-butyl or represent together butane-1,4-diyl (tetramethylene) or pentane-1,5-diyl (pentamethylene),
R³ represents hydrogen, methyl, ethyl, propyl, isopropyl, butyl, isobutyl or sec-butyl,
R⁴ represents hydrogen, methyl, ethyl, propyl, isopropyl, butyl, isobutyl, sec-butyl, tert-butyl, pentyl, isopentyl, sec-pentyl, tert-pentyl, hexyl, cyclopentyl, cyclohexyl, cyclopentylmethyl, cyclohexylmethyl, at any one time optionally substituted phenyl, benzyl or phenylethyl (the possible substituents in particular being chosen from the following: fluorine, chlorine, methyl, ethyl, trifluoromethyl, methoxy, ethoxy, difluoromethoxy, trifluoromethoxy), or
R³ and R⁴ represent, together with the nitrogen atom to which they are bound, pyrrolidinyl, piperidinyl, morpholinyl or piperazinyl, each of which is optionally substituted once to three times with methyl and/or ethyl, and
X represents fluorine, chlorine, methyl, ethyl, trifluoromethyl, methoxy, ethoxy, difluoromethoxy or trifluoromethoxy.

3. Process for the preparation of compounds of the formula (I) according to Claim 1, characterised in that
methylimidazolines of the general formula (II) 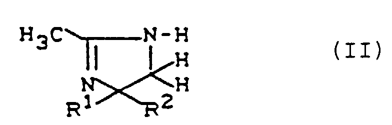 in which
R¹ and R² have the abovementioned meaning,
are reacted with phenylglyoxylamides of the general formula (III) 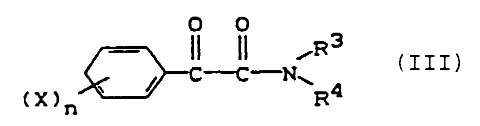 in which
n, R³, R⁴ and X have the abovementioned meaning,
optionally in the presence of a diluent and optionally in the presence of a basic catalyst.

4. Medicaments containing one or more compounds of Claims 1 and 2.

5. Antidiabetics containing one or more compounds of Claims 1 and 2.

6. Use of the compounds according to Claims 1 and 2 for preparing medicaments.

## Revendications

1. Composés de formule générale (I) 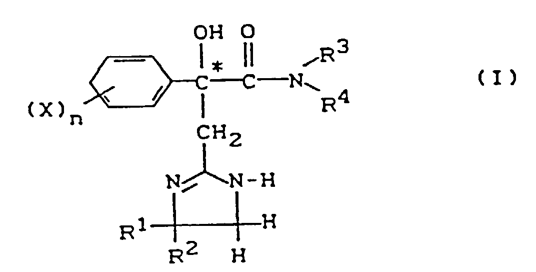 dans laquelle
n représente le nombre 0, 1, 2 ou 3,
R¹ et R² sont identiques ou différents et représentent, individuellement, l'hydrogène ou un groupe alkyle en C₁ à C₆ ou forment ensemble un groupe alcanediyle en C₄ à C₆,
R³ est de l'hydrogène ou un groupe alkyle en C₁ à C₆,
R⁴ représente de l'hydrogène, un groupe alkyle en C₁ à C₆ éventuellement substitué par du fluor, du chlore ou un reste alkoxy en C₁ à C₄, un groupe cycloalkyle en C₃ à C₇ ou un groupe (cycloalkyle en C₃ à C₇)-(alkyle en C₁ à C₄) dont chacun est éventuellement substitué par du fluor, du chlore, du brome ou un reste alkyle en C₁ à C₄, un groupe phényle, un groupe naphtyle, un groupe phényl-(alkyle en C₁ à C₄) ou un groupe naphtyl-(alkyle en C₁ à C₄) dont chacun est éventuellement substitué (les substituants possibles étant choisis de préférence dans la série comprenant le fluor, le chlore, le brome, un groupe cyano, nitro, un groupe alkyle en C₁ à C₄, un groupe alkoxy en C₁ à C₄, un groupe alkylthio en C₁ à C₄, un groupe alkylsulfinyle en C₁ à C₄ ou un groupe alkylsulfonyle en C₁ à C₄ dont chacun est éventuellement substitué par du fluor et/ou du chlore), ou bien
R³ et R⁴ forment, conjointement avec l'atome d'azote auquel ils sont liés, un hétérocycle pentagonal à heptagonal saturé ou non saturé éventuellement substitué une à trois fois par reste alkyle en C₁ à C₄ et qui contient, le cas échéant, un autre hétéroatome tel que de l'oxygène ou du soufre, et
x représente du fluor, du chlore, du brome ou bien un groupe alkyle en C₁ à C₄ ou un groupe alkoxy en C₁ à C₄ dont chacun est éventuellement substitué par du fluor et/ou du chlore.

2. Composés de formule (I) suivant la revendication 1, dans laquelle
n représente le nombre 0, 1 ou 2,
R¹ et R² sont identiques ou différents et représentent, individuellement, de l'hydrogène, un groupe méthyle, éthyle, propoyle, isopropyle, butyle, isobutyle ou sec.-butyle ou forment ensemble un groupe butane-1,4-diyle (tétraméthylène) ou pentane-1,5-diyle (pentaméthylène),
R³ est de l'hydrogène, un groupe méthyle, éthyle, propyle, isopropyle, butyle, isobutyle ou sec.- butyle,
R⁴ représente de l'hydrogène, un groupe méthyle, éthyle, propyle, isopropyle, butyle, isobutyle, sec.-butyle, tertio-butyle, pentyle, isopentyle, sec.-pentyle, tertio-pentyle, hexyle, cyclopentyle, cyclohexyle, cyclopentylméthyle, cyclohexylméthyle, un groupe phényle, un groupe benzyle ou un groupe phényléthyle dont chacun est éventuellement substitué (les substituants possibles étant choisis notamment dans la série du fluor, du chlore, des restes méthyle, éthyle, trifluorométhyle, méthoxy, éthoxy, difluorométhoxy, trifluorométhoxy), ou bien
R³ et R⁴ forment, conjointement avec l'atome d'azote auquel ils sont liés, un groupe pyrrolidinyle, un groupe pipéridinyle, un groupe morpholinyle ou un groupe pipérazinyle dont chacun est éventuellement substitué une à trois fois par un reste méthyle et/ou un reste éthyle, et
X représente du fluor, du chlore, un groupe méthyle, éthyle, trifluorométhyle, méthoxy, éthoxy, difluorométhoxy ou trifluorométhoxy.

3. Procédé de production de composés de formule (I) suivant la revendication 1, caractérisé en ce qu'on fait réagir, éventuellement en présence d'un diluant et en la présence éventuelle d'un catalyseur basique, des méthylimidazolines de formule générale (II) 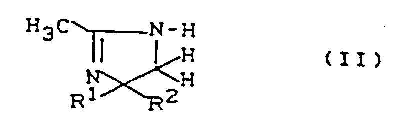 dans laquelle
R¹ et R² ont la définition indiquée ci-dessus, avec des phénylglyoxylamides de formule générale (III) 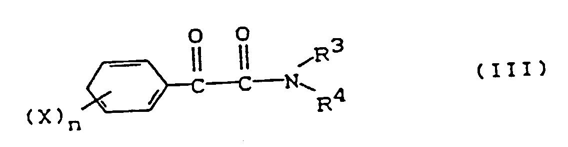 dans laquelle
n, R³, R⁴ et X ont la définition indiquée ci-dessus.

4. Médicaments contenant un ou plusieurs composés suivant les revendications 1 et 2.

5. Agents antidiabétiques contenant un ou plusieurs composés suivant les revendications 1 et 2.

6. Utilisation des composés suivant les revendications 1 et 2 pour la préparation de médicaments.
